# EUROPEAN PATENT APPLICATION

(11) **EP 3 750 527 A1**
(43) Date of publication of application: **16.12.2020**
(21) Application number: 19179999.8
(22) Date of filing: 13.06.2019
(51) Int. Cl.: A61K 9/20, A61K 31/541

(54) **STABLE TABLET FORMULATION OF NIFURTIMOX AND PROCESS FOR PRODUCING THE SAME**

(71) Applicant: Bayer AG, 51373 Leverkusen (DE)
(72) Inventor: The designation of the inventor has not yet been filed
(74) Representative: BIP Patents

(57) **Abstract**

The present invention is related to a new tablet formulation comprising nifurtimox and to a process for production thereof, to the use thereof as a medicament, and to the use thereof for prophylaxis and/or treatment of Chagas diseases.

## Description

The present invention is related to a new tablet formulation comprising nifurtimox and to a process for production thereof, to the use thereof as a medicament, and to the use thereof for prophylaxis and/or treatment of Chagas diseases.

Nifurtimox (3-Methyl-4-(5'-nitrofurfurylidene-amino)-tetrahydro-4H-1,4-thiazine-1,1-dioxide), shown below as formula (I), is used to treat Chagas disease. Chagas disease is caused by the protozoan parasite *Trypanosoma cruzi*, which is widely distributed throughout the Americas, particularly in poor, rural areas of Mexico, Central America, and South America.

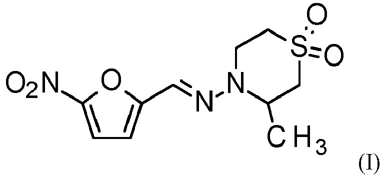

The Chagas disease, caused for the *Trypanosoma cruzi protozoa*, affects 18 million people in Latin America, what represents a local social responsibility of six billion dollar (WHO, Control of Chagas Disease. Technical Report Series 811, 1991). The decurrent morbidity and lethality of the Chagas disease are higher than that produced by the malaria, schistosomiasis and leishmaniasis (Schmuñis G.A., "Riesgo de la enfermedad de Chagas a través de las transfusiones en las Americas", Medicina (Buenos Aires) 1999; 59 (Suppl. II): 125-134). In Brazil, six million people are carriers of the disease.

The *T. cruzi* is transmitted to the mammals through triatomines and the infection occurs for the invasion of phagocytic cells, which can be transmitted for other individuals through blood transfusion or vertical transmission (from mother to son through the placenta). The pathological injuries found in the heart and digestive system of patients of chronic Chagas disease can be hidden for decades after the initial infection, not being exclusively explained for the destruction of the host tissue injured for the active parasite. The unit of minimum rejection, that consists of host target cells, free of parasite, lysed for mononuclear cells of the immunologic system, has been a common denominator of the Chagas disease pathology.

5-Nitro-furfurylidene-(1) derivatives, especially the compound according to formula (I) and the use of the compounds as anthelmintics for the treatment of *Trypanosoma cruzi*, were already disclosed in US 3,262,930.

Nifurtimox is available on the market as Lampit® tablets containing 120 mg active drug which were administered three times a day with the duration of treatment ranging from 90 days (acute infection) to 120 days (chronic infection).

A preferred administration route for nifurtimox is oral administration. As a consequence of the very low water solubility of nifurtimox (Biopharmaceutics Classification System class 2), it has only inadequate bioavailability after oral administration.

The so far available tablet formulation however shows still room for improvement with regard to oral dosage forms. For example the tablets show a low mechanical strength and tend to be hygroscopic during the production process, especially for the 30 mg dose strength.

There is still the need arose to provide nifurtimox also for the Pediatric Chagas Disease. To facilitate flexible dosing, especially for children, easily dividable scored tablets should have been developed in dose strengths of 30 mg and 120 mg. The new tablets should fulfill current requirements for immediate release tablets.

It was an object of the present invention to provide a new tablet formulation comprising nifurtimox which shows an increased mechanical stability and a faster immediate-release dissolution profile and could be prepared by roller compaction leading to a tablet with 30% drug load. Moreover, the tablets should be still easily dividable despite their higher mechanical strength and should fulfill dissolution specification for IR tablets.

Therefore it was also an object of the present invention to provide a tablet formulation of nifurtimox that allows manufacturing of a small tablet which is easy to swallow, easily dividable, fast to disintegrate and to release the active, while said tablet can reliably be manufactured. The resulting tablet is therefore also an aspect of the present invention.

Another object of the present invention was to provide a manufacturing process that is suitable to manufacture tablets out of the formulation according to the present invention that also addresses the mechanical strength and the sensitivity to moisture.

According to a first aspect of the present invention this is achieved with a tablet formulation comprising nifurtimox, at least one type of a dry binder, at least one disintegrant, at least one glidant and/or lubricant, characterized in that at least one type of disintegrant is selected from cross-linked polyvinylpyrrolidone or croscarmellose-sodium which has an average particle size between 145 to 25 µm, preferably 140 to 40 µm, more preferably 136 to 110 µm.

Moreover, it was found that the use of a dry granulation by roller compaction process instead of a wet granulation process of the tablets avoids the influence of moisture/humidity during the manufacturing process.

### Dry binder or filler

Preferred dry binders or fillers pursuant to the present invention are pharmaceutically acceptable excipients that are selected from the list consisting of microcrystalline cellulose, methylcellulose, hydroxypropylmethylcellulose, hydroxypropylcellulose and polyvinylpyrrolidone.

A very preferred dry binder is microcrystalline cellulose.

The dry binders are preferably present in the formulation in an overall amount of about 30% to 70% by weight, more preferably about 50% to 65% by weight, even more preferably about 60% to 64% by weight.

Addition of excipients with disintegration promoting properties such as microcrystalline cellulose, however further helps to improve the long time stability of the disintegration properties of tablets formed out of such formulations.

Formulations according to the present invention contain at least one disintegrant.

Disintegrants suitable in the context of the present invention are those selected from the list consisting of alginic acid, cross-linked polyvinylpyrrolidone, carboxymethylcellulose-derivatives such as carmellose calcium (carboxymethylcellulose calcium) and croscarmellose-sodium (cross-linked polymer of carboxymethylcellulose sodium), maize starch, modified starch, and starch derivatives such as sodium carboxymethyl starch.

Preferred disintegrants pursuant to the present invention are those selected from the list consisting of sodium starch glycolate (such as, for example, Explotab®), cross-linked polyvinylpyrrolidone (such as Polyplasdone®) and croscarmellose-sodium (such as, for example, AcDiSol®).

Very preferred disintegrants are cross-linked polyvinylpyrrolidones (such as Polyplasdone®) and/or croscarmellose-sodium (such as AcDiSol®).

Particularly preferred disintegrants are cross-linked polyvinylpyrrolidones (such as Polyplasdone®).

Those particularly preferred disintegrants being cross-linked polyvinylpyrrolidones are available at different grades, differentiated by particle size. If sold under the above referred to brand name "Polyplasdone®", three grades from coarse to fine can be discriminated. Those grades are Polyplasdone® XL, Polyplasdone® XL-10 and Polyplasdone® INF-10 and correspond - in the same order - to average particle sizes) of about 140 to 110 µm, about 40 to 25 µm and about 12 to 10 µm.

A particularly preferred formulation pursuant to the present invention comprises cross-linked polyvinylpyrrolidone Polyplasdone® XL-10 with an average particle size of about 40 to 25 µm as a disintegrant.

A further particularly preferred formulation pursuant to the present invention comprises cross-linked polyvinylpyrrolidone Polyplasdone® XL with an average particle size of about 140 to 110 µm as a disintegrant.

According to the preferred embodiment of the present invention, cross-linked polyvinylpyrrolidone or croscarmellose-sodium is present in the formulation in an amount of about 2.5% to 7.5% by weight, preferably in an amount of about 4% to 6%, more preferably in an amount of about 5% by weight.

According to the particularly preferred embodiment of the present invention, cross-linked polyvinylpyrrolidone with an average particle size of about 140 to 110 µm in an amount of about 2.5% to 7.5% by weight, preferably in an amount of about 4% to 6%, more preferably in an amount of about 5% by weight.

Furthermore formulations according to the present invention contain at least one lubricant and/or glidant (flow agent).

Lubricants that can be used in the formulation according to the present invention are those selected from the list consisting of magnesium stearate, sodium stearylfumarate, stearic acid, glycerin monostearate, glycerin monobehenate, calcium behenate, hydogenated vegetable fat or oil, polyethylenglycol or talc.

Preferred lubricants according to the present invention are those selected from the list consisting of magnesium stearate, stearic acid or talc. A very preferred lubricant is magnesium stearate.

A glidant (flow agent) is an excipient that improves the flow characteristics of a compressible powder such as tablet ingredients or granules. Two of the most common glidants are colloidal silicon dioxide (CAB-O-SI®) and Quso (also known as Phila Quartz). A preferred glidant used in the formulation according to the present invention is highly-dispersed silica (for example Aerosil®).

If used in combination, a preferred combination of lubricant and glidant is magnesium stearate and highly-dispersed silica.

In a preferred embodiment of this invention the formulation comprises at least one glidant and at least one lubricant.

In a further preferred embodiment of this invention the formulation only comprises a lubricant, which preferably is magnesium stearate.

Preferred amounts of lubricants present in the formulation according to the present invention are amounts of about 0.1% to 3% by weight, preferably amounts of about 0.5% to 1.5%, more preferably amounts of about 1% by weight.

If present, preferred amounts of flow agents are amounts of about 1% to 0.1% by weight, more preferably amounts of about 0.75% to 0.25%, even more preferably amounts of about 0.5% by weight.

According to a likewise preferred embodiment of the present invention the glidant is highly-dispersed silica present in the formulation in an amount of about 0.1% to 1% by weight, preferably in an amount of about 0.25% to 0.75%, more preferably in an amount of about 0.5% by weight, and the lubricant is magnesium stearate present in the formulation in an amount of about 0.1% to 3% by weight, preferably in an amount of about 0.5% to 1.5%, more preferably in an amount of about 1% by weight.

The overall tablet size can be kept of less than or equal to 10 mm, preferred between 7 mm and 10 mm.

In an preferred embodiment of the present invention, the formulation comprises
- 30% by weight of nifurtimox,
- 30% to 70% by weight, preferably 50% to 65% by weight, more preferably 60% to 65% by weight of microcrystalline cellulose,
- 2.5% to 7.5% by weight, preferably 4% to 6%, more preferably 5% by weight of a disintegrant,
- 0.11% to 1% by weight, preferably 0.25% to 0.75%, more preferably 0.5% by weight of a glidant, and
- 0.1% to 3% by weight, more preferably 0.5% to 1.5%, even more preferably about 1% by weight lubricant.

In a more preferred embodiment of the present invention, the formulation comprises
- about 30% by weight of nifurtimox,
- about 63.5% by weight of microcrystalline cellulose,
- about 5% by weight of a disintegrant,
- about 0.5% by weight of a glidant, preferably highly-dispersed silica and
- about 1.0% by weight of a lubricant, preferably magnesium stearate.

As anyone in the field of pharmaceutical formulation technology may be aware of, the above referred to functional descriptions of being a 'dry binder', 'disintegrant', 'glidant', 'flow agent' and 'lubricant' may be assigned to substances depending on their intended purpose in a given formulation. All of the above types of substances are summarized under the genus of being 'excipients' together with the 'fillers' described below.

It may therefore come to happen that one of ordinary skill in the art assigns similar or even identical substances to be member of more than one of the above referred to groups of substances. Within the context of the present invention, the functional descriptions of the substances are however intentionally filled with specific substances to have no overlap to clarify their respective property assigned to them.

However, any formulation according to the present invention may additionally comprise any further combination of pharmaceutically suitable excipients as described above.

Such excipients can also be fillers as commonly known in the art, which are organic fillers or inorganic fillers. Among the organic fillers, those fillers can be sugars or sugar alcohols such as mannitol as well as any carbohydrates such as maltodextrine.

Among the inorganic fillers, those fillers can be for example calcium hydrogenphosphate or calcium carbonate or any mixtures of the aforementioned organic and/or inorganic fillers thereof.

The tablets produced out of a formulation pursuant to the preferred embodiments of the present invention combine all beneficial effects that have been referenced before.

The dosage form of a tablet is generally described in literature (European Pharmacopoeia 9.0; Chapter 7 - Dosage forms - Tablets). Within the context of the present invention a tablet is - if not mentioned otherwise - the uncoated core of a tablet. Particularly, it represents a formulation pursuant to the present invention, pressed into the form of a tablet.

However, the tablet could be optionally coated with a functional coating or a coating layer that comprises or consists essentially of a coating agent that is insoluble in water, but soluble in aqueous media with a pH below 5, preferably a pH below 4. This functional coating is applied by inventional coating techniques, such as pan coating or fluid bed coating using solution, suspension or dispersion of polymeric material in water or suitable organic solvents.

The tablets containing the formulation according to the present invention, form a second aspect of the present invention.

With regard to disintegration, this is to be understood pursuant to the present invention to be a disintegration time measured pursuant to European Pharmacopoeia 9.2 (2017) section 2.9.1 Disintegration of tablets and capsules. Obviously a shorter time is indicative for a faster disintegration and a longer time is indicative for inferior disintegration.

Tablets containing the formulation pursuant to the first embodiment as described just above within this invention, share the positive effect of collectively having a sufficient disintegration time of less than 1 min, preferably less than 30 sec. Tablets containing the formulation according to the invention, share the positive effect of collectively having a sufficient mechanical strength with resistance to crushing values of 65 N to 70 N for the 30 mg nifurtimox tablet dose strength and 100 N for the 120 mg nifurtimox tablet dose strength.

Tablets pursuant to the present invention may be in whatever kind of form that is deemed to be advantageous.

In a fourth aspect of the present invention, a process for the production of a tablet according to the second aspect of the invention is provided, wherein said process comprises the steps of
a) dry mixing nifurtimox together with at least one dry binder and at least one disintegrant,
b) dry granulating the resulting mixture to obtain dry granules,
c) at least once blending said dry granules with at least one flow agent and/or lubricant to obtain the ready-to-press blend,
d) tableting said blend to become a tablet.

Within the process of the fourth aspect of the present invention, it is preferred that the dry granulation performed pursuant to step b) of the process is done as a roller compaction.

It has surprisingly been found that the above referred process allows to produce tablets of the nifurtimox formulation without having to add water. It was found that dry granulation instead of wet granulation can be used, which is particularly advantageous, as such process addresses the (negative) hygroscopic properties of nifurtimox.

Furthermore the above process is capable to produce tablets in high amounts in continuous manufacturing, which comes along with meeting the above referred to objective to provide tablets to a huge market at affordable prizes.

### Examples

### Process of producing tablets from formulations pursuant to the present invention

Excipients (see below) were blended with nifurtimox to the extent exemplified below. Afterwards these powder blends were compacted with an instrumented roller compactor and grinded afterwards.

Subsequently the granules were blended with the flow agent and/or the lubricant. This ready-to-press blend was compressed on a rotary die press as well as on a compression simulator to round tablets of different sizes 7 mm (30 mg), 10 mm (120 mg) and masses of 100 mg (30 mg) and 400 mg (120 mg).

### Assessment of the tablets on dissolution/disintegration

Disintegration experiments were performed according to European Pharmacopoeia 9.2 (2017) section 2.9.1 Disintegration of tablets and capsules using a disk. The mean of the measurement times was calculated.

The dissolution experiments were performed according to the USP 40 (chapter <711> Dissolution) using apparatus 2 (paddle method). Stirrer speed was 100 rpm, release medium phosphate buffer pH 4.5 + 1.0 % SLS (for the 30 mg dose strength) or + 1.5 % SLS (for the 120 mg dose strength) and temperature 37 °C.

For disintegration and dissolution experiments 6 tablets were tested.

| **Composition** | **Function** | **Amount [mg]** |
|---|---|---|
| | | |

| **Drug substance** | | |
|---|---|---|
| Nifurtimox micronized | drug substance | 30.00 |
| | | |

| **Excipients** | | |
|---|---|---|
| Cellulose microcrystalline | dry binder | 63.50 |
| Crospovidone | disintegrant | 5.00 |
| Silica colloidal anhydrous | glidant | 0.50 |
| Magnesium stearate | lubricant | 1.00 |
| | | |
| **Weight** | | **100.00** |

## Claims

1. Tablet formulation comprising nifurtimox, at least one type of a dry binder, at least one disintegrant, at least one glidant and/or lubricant, **characterized in that** at least one type of disintegrant is selected from cross-linked polyvinylpyrrolidone or croscarmellose-sodium which has an average particle size between 145 to 25 µm.

2. Tablet formulation according to Claim 1, **characterized in that** disintegrants are cross-linked polyvinylpyrrolidones and/or croscarmellose-sodium.

3. Tablet formulation according to Claim 2, **characterized in that** the cross-linked polyvinylpyrrolidone has an average particle size of about 140 to 110 µm.

4. Tablet formulation according to Claim 2, **characterized in that** the cross-linked polyvinylpyrrolidone has an average particle size of about 40 to 25 µm.

5. Tablet formulation according to Claim 3, **characterized in that** formulation contains the cross-linked polyvinylpyrrolidone with an average particle size of about 140 to 110 µm in an amount of about 2.5% to 7.5% by weight.

6. Tablet formulation according to Claim 1, **characterized in that** the formulation comprises
- 30% by weight of nifurtimox,
- 30% to 70% by weight of microcrystalline cellulose,
- 2.5% to 7.5% by weight of a disintegrant,
- 0.1% to 1% by weight of a glidant, and
- 0.1% to 3% by weight of a lubricant.

7. Tablets containing the formulation according to any of Claims 1 to 6.

8. Process for the production of a tablet according to Claim 7, wherein the process comprises the steps of
a. dry mixing Nifurtimox together with at least one dry binder and at least one disintegrant,
b. dry granulating the resulting mixture to obtain dry granules,
c. at least once blending said dry granules with at least one flow agent and/or lubricant to obtain the ready-to-press blend,
d. tableting said blend to become a tablet.

9. Process according to Claim 8, **characterized in that** the dry granulation performed pursuant to step b) of the process is done as a roller compaction.

10. Medicament, comprising a tablet formulation according to any of Claims 1 to 6.

11. Use of a tablet formulation according to any of Claims 1 to 6 for preparing a medicament for the prophylaxis and/or treatment of Chagas disease.
